# EUROPEAN PATENT APPLICATION

(11) **EP 1 647 276 A1**
(43) Date of publication of application: **19.04.2006**
(21) Application number: 04024440.2
(22) Date of filing: 14.10.2004
(51) Int. Cl.: A61K 31/549, A61P 31/04

(54) **Use of taurolidine formulations for the intramammary treatment of mastitis**

(71) Applicant: BOEHRINGER INGELHEIM VETMEDICA GMBH, 55216 Ingelheim am Rhein (DE)
(72) Inventor: Muellner, Hubert, 65779 Kelkheim (DE); Daemmgen, Juergen, 88416 Ochsenhausen (DE); Kron, Alexander, 19057 Schwerin (DE); Folger, A. Martin, 55218 Ingelheim (DE)

(57) **Abstract**

The invention is directed to the use of a formulation containing taurolidine and one or more vehicles for preparing a veterinary medical composition for intramammary treatment of mastitis or the prevention thereof. The intramammary administration leads to an effective concentration in the target tissue, which is achieved very quickly. The taurolidine formulation used according to the present invention is the first product for the treatment of clinical, subclinical and chronic mastitis without residues, without a significant withdrawal period and without the resistance induction image of classical antibiotics.

## Description

### FIELD OF THE INVENTION

The present invention is directed to the novel use of taurolidine formulations in veterinary medicine, especially for the treatment of mastitis in agricultural animals such as dairy cows.

### BACKGROUND OF THE INVENTION

The inflammation of the udder (mastitis) is one of the most important diseases in dairy cattle. Promoted by a higher milk production said disease results in significant losses to dairy industry and increased culling rates. In most cases insufficient hygienic provisions and increasing susceptibility of the animals suffering under high-perfomance conditions lead to a subclinical inflammation of the udder primarily initiated by gram(+) pathogens such as staphylococcus and streptococcus. In contrast, however, the clinical mastitis or the acute udder inflammation is frequently initiated by gram(-) pathogens. Mastitis-causing pathogens are, for example, contagious pathogens that are associated with the udder (i.e. Staphylococcus aureus, Streptococcus agalactiae, Streptococcus dysgalactiae and coagulase-negative Staphylococci) or environmental pathogens that are present in a cow's environment (coliform bacteria and streptococci other than Streptococcus agalactiae).

The infection may be clinically obvious with severe signs of illness or subclinically with no obvious signs. General clinical signs refer to increased rectal temperature (up to high fever) and severe deterioration in a cow's general state of health (no or reduced feed intake, impaired general condition). Local clinical signs include changes in macroscopic milk quality associated with typical inflammatory reactions of the affected quarter (red, swollen, warm and painful). The infection may be recognized with an increased number of immunological cells in the milk. A high number of immunological cells in the milk is an indication for an infection even if no bacteria may be detected. The extent of udder infection in a dairy cow is therefore usually assessed through measuring the number of somatic cells present in milk.

If the subclinical mastitis is not treated a chronical mastitis will occur by-and-by and the resulting modification of the gland tissue of the udder may lead to a substantially reduction of milk production and therefore to a financial loss of the dairy farm. As a result, during lactation or the dry cow period antibiotics are used either systemically or intramammarily in dairy cattle for the treatment of chronic mastitis.

Antibiotics are commonly used for treatment and prevention of infections of the udder and various products are available. Although new antibiotic presentations continue to be launched, animal health companies and the dairy industry are increasingly looking towards alternatives for treatment, metaphylaxis and prophylaxis of mastitis. Antibiotics are usually administered by intramammary application and in severe cases of infection, they may be administered parenterally in addition. Intramammary preparations are supplied in disposable single-use syringes or injectors.

However, antibiotics are drugs which involve or may be associated with undesirable side effects. For example, hypersensitive reactions have been found when antibiotics are used in human medicine. In the veterinary medical field, in particular, the administration of large quantities of antibiotics to animals which are intended for consumption, or the products of which are intended for consumption, may lead to long waiting times to ensure that the drugs are not unintentionally taken by humans and thus promote the build-up of resistance to the pathogens, for example.

Due to the problems related with the formation of resistance against antibiotics and the thesis that the use of antibiotics in the keeping of animals should create an increased resistance against human pathogenes, the administration of antibiotics is coming more and more under pressure. Products which have been proved not to contribute to an aggravation of the problems related with resistance may be preferred in case they show an equivalent or similar spectrum of action over a classical antibiotic agent.

A known active agent is taurolidine (trademark Taurolin® ), a bicyclic derivative of the aminosulfonic acid taurine produced by reaction of two molecules taurine with three molecules of formaldehyde. The chemical name is bis-(1,1-dioxoperhydro-1,2,4-thiadiazynol-4)-methane and the chemical formula is as follows:

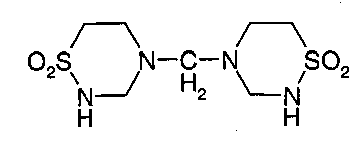

The mechanism of action is the chemical reaction or cleavage of the bicyclic molecule taurolidine with activated amino groups and hydroxyl groups, which are present on the surface of microorganisms, bacteria, fungi and viruses or in endo/exotoxin molecules or tumor cells of mammals. In the course of this process taurolidine completely disintegrates in three steps to taurinamide and taurine. Crosslinked surface structures and damaged membrane processes will occur, resulting in the death of the cells.

Taurine is an ubiquitary substance in the human and animal body and is responsible to accomplish a series of tasks in brain, heart and liver (J. A. Sturman, G. W. Hepner et al., "Taurine", 1976, pp. 21-33, editor R. Huxtable and A. Barbeau, Raven Press, New York).

Actually, taurolidine is known for more than 30 years and was firstly synthesized in 1966. In the recent decades numerous publications documented and described a variety of effects of taurolidine on microorganism and cells of mammals (Ed. W. L.

Brückner and W. Pfirrmann, "Taurolin - Eine neues Konzept zur antimikrobiellen Chemotherapie chirurgischer Infektionen"; 1985, Urban & Schwarzenberg, München; C. A. Jakobi, "Taurolidin in der Tumortherapie - Vorstellung eines Therapiekonzeptes", Unimed Science, Uni-Med Verlag AG, Bremen - London - Boston, 2003). Primarily the wide bactericidal, fungicidal and virucidal activity may be mentioned, resulting in the use as adjuvant therapy in case of inflammations in the abdominal area, e.g. peritonitis (P. Auckland, J. Wakeley, "Local Taurolin in severe infective peritonitis"; 1985; Ed. W. L. Brückner and W. Pfirrmann, ibid.).

Therefore, taurolidine is used in the human pharma market, for example, for the treatment of peritonitis, particularly rinsing of the abdominal area after a surgery, or instillation into the abdomen where it remains. Numerous other applications are documented e.g to influence wound healing, to reduce catheter clogging by biofilm generating microorganisms, to cure otitis, to assist in soft tissues and bone injuries, it has an inhibitory effect on leukaemia cells, it can reduce the attachment of metastatic cells in the peritoneum after surgery of colon carcinoma and it drives cancer cells into apoptosis. In the recent years the administration of taurolidine was of increasing importance as adjuvant therapy in tumor resections of the gastrointestinal tract as well as other applications in the human pharma sector, such as the oncology field as anti microbial chemotherapeutic, but applications in the field of animal health have hardly been documented.

The only scientific paper found is a PhD thesis of 1985 (Jean-Christophe Dubuis, "Antitoxicite de la Taurolin et de la polymyxine testée sur un modèle experimental de mammite coliforme induite par une endotoxine d'Escherichia coli", These inaugurale of Dissertation, University of Bern; cf. Schweizer Archiv für Tierheilkunde, Vol. 127, No. (7), pp. 450, 1985) wherein an instillation of taurolidine into the udder has been described. The objective, however, was not to provide a therapy of an udder inflammation but to evaluate an additional systemic application for an improved protective action and control against endotoxines, but no effect on the applied endotoxins could be reached and the bad compliance of the product used prevents that further experiments were conducted. A further PhD thesis of 1996 (Barbara Knutti, "Intrauterine Behandlung der Endometritis beim Rind mit Taurolin", Inaugurale Dissertation, University of Bern) shows a comparable action of taurolidine over a reference product for the treatment of metritis in cattles.

However, a compatible and effective therapy for udder inflammation with taurolidine is not described up to now. No patents, patent applications or scientific publications describe the use of taurolidine to treat successfully mastitis.

Therefore, it is an object of the present invention to provide a pharmaceutical formulation for the treatment and prevention of mastitis, clinical and subclinical cases, but particularily chronic cases, which makes it possible to allow an effective and economic control of udder infections. The disadvantages of a therapy with antibiotics should be avoided but an activity spectrum which is equal or similar to that of an antibiotic should be achieved.

### DESCRIPTION OF THE INVENTION

Surprisingly, it has been found that taurolidine formulations may be used for intramammary treatment of mastitis cases in order to reduce local clinical signs of inflammation in the udder and to relief pain.

Therefore, the present invention provides the use of a formulation containing taurolidine, one or more vehicles, and optionally one or more suitable additives for preparing a veterinary medical composition for intramammary treatment of mastitis or for the prevention thereof.

The taurolidine containing formulation may be used directly on the udder or may be applied into the udder without showing the negative effects provided by antibiotics and resulting in excellent healing effects.

Actually, the use of the taurolidine formulation of the present invention leads to a decrease of the local inflammation symptoms if introduced through the streak canal in a quarter of the udder suffering from a microbially induced disease such as a mastitis infection. The taurolidine formulation used according to the invention exhibits a local anti-inflammatory effect, leading to a clinical improvement as well as a reduction of the concentration of the inflammatory mediators.

It has been found that taurolidine controls gram(+) and gram(-) pathogens as well as fungi. Taurolidine blocks coagulase from Staph. aureus and reduces inflammation indirectly by lowering the release of Interleukin 1 beta and Tumor necrosis factor alpha. Furthermore the active substance used according to the present invention inhibits the attachment of bacteria to cell walls. In the body of an animal taurolidine has a half life of about 4 hours and will degrade to taurine a normal body constituent. Moreover no toxic residues occur in the mechanism of action and the related metabolism and a very short withdrawal period of one day or less has been found.

Additionally taurolidine, in contrast to all other mastitis therapeutics, is not an antibiotic and it will not induce resistance. Taurolidine does not lead to the developement of resistance due to its mechanism of action. The wide range of activity comprising gram(+) and gram(-) pathogens, the specific action on staphylococcus (inhibition of coagulase) and the comparatively rapid decomposition in the ubiquitary body constituent taurine, i.e. low waiting times, makes the application of taurolidine suitable for the therapy of mastitis which is in contrast to the intramammary use in 1985, the above-mentioned PhD in dairy cattle, which allegedly has shown explicit symptoms of incompatibility.

According to the present invention it is now established that taurolidine is a broad spectrum product without the typical problems of classical antibiotics like the possibility of resistance induction and undesired residues in edible tissue. The taurolidine formulation of the present invention is the first product for the treatment of clinical, subclinical and chronic mastitis without critical residues, without a significant withdrawal period and without the resistance induction known from classical antibiotics.

According to a preferred embodiment the formulation according to the present invention contains taurolidine in a concentration of 1 to 300 mg/ml, preferably 5 to 250 mg/ml, more preferably 10 to 200 mg/ml, particularly preferred 15 to 150 mg/ml, especially preferred 20 to 100 mg/ml, especially 25 to 35 mg/ml.

Furthermore it is also possible to use a taurolidine formulation as available on the market such as a taurolidine 2 % aqueous solution with polyvinyl pyrrolidone as stabilizer/solubiliser or as a taurolidine 0.5 % Ringer solution both provided by Geistlich Pharma, Wolhusen, Schweiz. The available formulations may be used in dairy cows, but the formulations may also be optimized for the treatment of mastitis in cattle.

The taurolidine containing formulation used in the present invention may contain, in addition to the active substance, one or more vehicles and optionally one or more additives. Depending from the vehicle the other additives may be selected accordingly. The vehicle may be selected from water and/or oil to result in an aqueous or oily system; intermediate systems are also possible.

In the frame of the present invention the expression "solution" should be understood to comprise dispersed systems, true solutions as well as intermediate systems.

If an aqueous system is selected Ringer's Lactate solution can be used as a suitable vehicle. Ringer's solution, also known as Ringer's irrigation, is an aqueous solution of the chlorides of sodium, potassium, and calcium that is isotonic to animal tissue and is used topically as a physiological saline and, in experiments, to bath animal tissues - in practice nearly the same concentrations are used as their occurrence in body fluids. Ringer's lactate solution additionally contains a lactate.

The aqueous system may for example optionally contain a suitable gelling agent and/or a viscosity enhancer leading to a viscous aqueous solution or a hydrogel.

If an oily system is selected the additives may preferably be selected from one or more oils, optionally one or more antioxidants, and optionally one or more thickeners. It is a matter of course that also other additives may be present.

Suitable formulations are preparations for intramammary use (in accordance with the requirements of e.g. Ph. Eur.), containing the active substance taurolidine in a very low concentration of for example about 0.1 to several percent, for example about 2 %.

Suitable formulations are preparations for intramammary use (in accordance with the requirements of e.g. Ph. Eur.) which preferably contain or essentially consist of taurolidine and Ringer's lactate solution as a suitable vehicle. The aqueous system may further contain one or more buffer and one or more thickener.

Suitable formulations are preparations for intramammary use (in accordance with the requirements of e.g. Ph. Eur.) which preferably contain or essentially consist of taurolidine, one or more oils, optionally one or more antioxidants and optionally one or more thickeners.

Some formulations by way of example follow the experimental section.

The formulation used according to the invention is suitable for treating mastitis, also clinical and subclinical cases, particularly chronic mastitis. It is suitable for treating mammals, particularly working animals or farm animals, such as dairy cattle.

Surprisingly it is observed that preferably one single dose per day, for example in an interval of 24 hours, or administred several times, for example in intervals of 8 hours and three times per day, may be tolerated in cows without side effects.

The dosage of the formulation according to the invention, either aqueous or oily system, should preferably correspond 200 to 2000 mg of active substance per udder quarter and day, more preferably 300 to 1500 mg per udder quarter and day, most preferably 400 to 1200 mg per udder quarter and day. For example the application of 20 ml of an 2 % aqueous solution of taurolidine (Taurin® 2%) which may preferably be stabilised with polyvinyl pyrrolidone (PVP) as available on the market may be used as single dose.

The formulation according to the invention may be prepared using the methods of preparing formulations known from the literature. For example, the appropriate additives may be added to a taurolidine preparation.

The taurolidine containing formulation of the invention may be administered in the form of an aqueous solution, oily or aqueous suspension, hydrogel, cream, ointment, lotion, water-in-oil emulsion, oil-in-water emulsion, aerosol foam or injector formulation. The preparation of pharmaceutical forms of this kind is well-known per se from the prior art.

Preferably use is made of an injector formulation. An injector comprises means which allows to inject the formulation intramammary, i.e. through the streak canal into the udder, with the formulation being present in a casing, reservoir, phiole, syringe or tube or the like, which may be disposable and provided for a single-use, containing a delivery system such as a suitable opening, channel or a blunt needle. This form of intramammary application may achieve a good distribution in the target organ together with an increase in the activity.

The advantages of the present invention are manifold:
The formulation according to the invention containing the active substance taurolidine provides a novel intramammary use for the treatment of mastitis.

Although the udder tissue is a highly sensitive tissue the use of the taurolidine formulation of the present invention has a good compliance and does not result in a local weakening of the body's own defences of the treated animal. This finding being in contrats to the results in prior art according to which taurolidine should not be suitable to be applied intramammary because there should occur no effect but a tissue irritation leading to a worsening of the disease.

On the contrary, the use of the taurolidine formulation of the present invention introduced in a quarter of the udder suffering from a mastitis infection, leads to a decrease of the local inflammation symptoms. The taurolidine formulation exhibits a local anti-inflammatory effect, which results in a clinical improvement. Furthermore, the intramammary administration leads to an effective concentration in the target tissue, which is achieved very quickly.

Furthermore, the taurolidine containing formulation according to the present invention controls gram(+) and gram(-) pathogens as well as fungi, blocks coagulase from Staph. aureus and reduces inflammation indirectly by lowering the release of Interleukin 1 beta and Tumor necrosis factor alpha. It inhibits the attachment of bacteria to cell walls. In the body of an animal the active substance taurolidine will degrade to taurine, a normal body constituent. No toxic residues and a very short withdrawal period of one day or less support the superior effectivity and the economic therapy thereof.

Furthermore it is also possible to use taurolidine in the forms as available on the market.

The taurolidine formulation used is a broad spectrum product without the typical problems of classical antibiotics like the possibility of resistance induction and the presence of undesired residues in edible tissue because taurolidine, in contrast to all other mastitis therapeutics, is not an antibiotic. Consequently, the non-development of a resistance, the wide range of activity and the comparatively rapid decomposition in the ubiquitary body constituent taurine connected with low waiting times, makes the application of taurolidine particularly suitable for the therapy of mastitis.

Actually, the taurolidine formulation used according to the present invention is the first product for the treatment of clinical, subclinical and chronic mastitis without residues, without a significant withdrawal period and without the resistance induction image of classical antibiotics.

The invention described will now be illustrated by the Examples which follow various other embodiments and will become apparent to the skilled person from the present specification. However, it is expressly pointed out that the Examples and description are intended solely as an illustration and should not be regarded as restricting the invention.

### Examples

In the following 4 formulations according to the present invention were prepared for intramammary use (in accordance with the requirements of Ph. Eur.) containing taurolidine in an aqueous or oily system. The formulations are listed in the following tables 1 to 4.

### Example 1:

Formulation 1 of the invention was prepared in form of an aqueous injector solution.

**Table 1**

| **ingredient** | **g/100 ml** |
|---|---|
| Taurolidine | 0.500 |
| Sodium chloride | 0.600 |
| Potassium chloride | 0.040 |
| Calcium chloride dihydrate | 0.027 |
| Sodium lactate solution 50 % (m/m) | 0.610 |
| Water for injection | ad 100 ml |

### Example 2:

Formulation 2 of the invention was prepared in form of an aqueous injector solution.

**Table 2**

| **ingredient** | **g/100 ml** |
|---|---|
| Taurolidine | 2.000 |
| Sodium chloride | 0.600 |
| Potassium chloride | 0.040 |
| Calcium chloride dihydrate | 0.027 |
| Sodium lactate solution 50 % (m/m) | 0.610 |
| Water for injection | ad 100 ml |

### Example 3:

Formulation 3 of the invention was prepared in form of an aqueous injector solution.

**Table 3**

| **ingredient** | **g/100 ml** |
|---|---|
| Taurolidine | 0.500 |
| Sodium chloride | 0.600 |
| Potassium chloride | 0.040 |
| Calcium chloride dihydrate | 0.027 |
| Sodium lactate solution 50 % (m/m) | 0.610 |
| Carboxymethylcellulose Sodium | 5.000 |
| Sodium hydroxide | q.s. to give pH 7 to 9 |
| Water for injection | ad 100 ml |

### Example 4:

Formulation 4 of the invention was prepared in form of an oily suspension.

**Table 4**

| **ingredient** | **g/100 ml** |
|---|---|
| Taurolidine | 2.000 |
| Aluminium monostearate | 2.000 |
| Alpha Tocopherol | 0.050 |
| Sesame Oil | ad 100 ml |

## Claims

1. Use of a formulation containing taurolidine and one or more vehicles for preparing a veterinary medical composition for intramammary treatment of mastitis or the prevention thereof.

2. Use of a formulation according to claim 2, **characterised in that** the content of taurolidine being from 1 to 300 mg/ml, preferably from 10 to 200 mg/ml, particularly about 30 mg/ml.

3. Use of a formulation according to claim 1 or 2, **characterised in that** the vehicle is selected from water and/or oil.

4. Use of a formulation according to claims 1 to 3, **characterised in that** the vehicle is Ringer's lactate solution.

5. Use of a formulation according to one of the preceding claims 1 to 4, **characterised in that** one or more suitable additives are present.

6. Use of a formulation according to one of the preceding claims 1 to 5, **characterized in that** it contains or essentially consists of taurolidine, Ringer's lactate solution, optional one or more buffer and optionally one or more thickeners.

7. Use of a formulation according to one of the preceding claims 1 to 6, **characterized in that** it contains or essentially consists of taurolidine, one or more oils, optionally one or more antioxidants and optionally one or more thickeners.

8. Use of the formulation according to one of the preceding claims 1 to 7, which corresponds to a dosage range of from 200 to 2000 mg of active substance, preferably 400 to 1200 mg of active substance per quarter udder and day.

9. Use of a formulation according to one of the preceding claims 1 to 8, **characterised in that** the formulation may be administered in the form of an aqueous solution, oily or aqueous suspension, hydrogel, cream, ointment, lotion, water-in-oil emulsion, oil-in-water emulsion, aerosol foam or injector formulation.
